# EUROPEAN PATENT APPLICATION

(11) **EP 1 882 744 A1**
(43) Date of publication of application: **30.01.2008**
(21) Application number: 07747858.4
(22) Date of filing: 16.03.2007
(51) Int. Cl.: C12P 21/02, C07K 14/55

(54) **METHOD FOR PRODUCING AN INTERLEUKIN-2 PREPARATION AND THE THUS OBTAINABLE PREPARATION**

(30) Priority: 27.03.2006 RU 2006110508
(71) Applicant: Smirnov, Mikhail Nikolaevich, St. Petersburg 191025 (RU)
(72) Inventor: NIKOLAEVA, Zoya Kalenikovna, St.Petersburg, 190000 (RU); YAKOVLEVA, Vera Sergeevna, St.Petersburg, 191014 (RU)
(74) Representative: Glawe, Delfs, Moll
(86) International application number: PCT/RU2007/000128
(87) International publication number: WO 2007/111534

(57) **Abstract**

A method for the preparation of interleukin-2 (IL-2) formulation is provided, wherein IL-2 producer yeast cells, that synthesize the target protein (IL-2) and do not secrete said target protein, are disrupted and target protein integrity is ensured and wherein water insoluble fraction is separated and water insoluble compounds of disrupted cells are preserved in the formulation. Preferably, yeast producer is a strain of *Saccharomyces cerevisiae,* deposited in the All-Russian Collection of Industrial Microorganisms as No. Y-791 that is a producer of human interleukin-2. The formulation of the invention has lower productivity cost compared to the well-known ones and is active when administered orally. In some cases this formulation is more active than formulations based on IL-2 and purified from components of yeast walls fraction.

## Description

The invention relates to the preparation of immunomodulating drugs by biotechnological methods and can be used in pharmaceutical industry. In particular, the present invention relates to the method of the preparation of interleukin-2 formulation. Said formulation can be used for immune-oriented therapy and, in particular, for treatment of oncological, infectious, autoimmune and allergic diseases.

Discovery of Interleukin-2 (IL-2) dates back to 1960^{th} when numerous studies in vitro showed that activated lymphocytes secrete mediator that stimulates a great number of immune reactions in vitro and in vivo. Only in 1976 this mediator was identified as T-cell growth factor named Interleukin-2 in 1979. Special technologies for producing and purifying IL-2 from culture of human activated lymphocytes have been developed, that allowed to obtain first preparations of IL-2 for scientific and clinical purposes. Those formulations were named "lymphocytic" or "natural" IL-2. At the same time, limited possibilities of scale-up of its manufacturing hindered its applying until 1983, when the technologies for generating recombinant proteins were developed, in particular, IL-2, by genetic engineering and biotechnology methods.

Methods of the preparation of IL-2 formulations from donor blood are also known. However, a formulation containing components of human blood is potentially hazardous.

Genetic engineering methods of the preparation of IL-2 are also known. Numerous companies (Cetus Corp., Amgen, Inc., Hoffman - La Roche, Inc.) have used methods of genetic engineering and biotechnology to produce recombinant IL-2 as a drug for treatment of various types of oncological diseases.

In one of the methods of the preparation of IL-2 *Escherichia coli* are used as producers. *Escherichia coli* are opportunistic flora. Therefore, formulations prepared from *E.coli* must be thoroughly purified and can be toxic.

Yeast cells are more attractive producers of heterologous IL-2.

Method of the preparation of IL-2 formulation using yeast producer (EP 0162898 A1, published on 12/ 04/1985) is known. According to this method, IL-2 is secreted into the culture medium and then it is necessary to purify it from the culture medium by means of chromatography, filtration, extraction, etc. Disadvantage of said method lies in the fact that proteins secreted by yeasts are glycosylated, i.e. yeast carbohydrate chains appear in their structure during secretion. Such glycoproteins are highly immunogenic for humans, which becomes a problem for their subsequent use in therapy, for example, accumulation of antibodies to such glycoproteins in humans.

Besides that, method (EP 0152358, published on 08/21/1985) of the preparation of IL-2 formulation is also known, wherein yeast cells in which IL-2 synthesis has been carried out, are disrupted, disrupted cells are centrifuged, sediment obtained after centrifugation is treated with solution of sodium dodecyl sulfate to transfer IL-2 into a liquid phase, and IL-2 is extracted from the liquid phase. Extraction of IL-2 in this method is a labour-intensive operation and is very time-consuming, requires special equipment and/or reagents.

On the whole, all recombinant IL-2 formulations known up to date are extremely expensive, and a significant contribution to their cost is made by technology of their purification from cell producers.

The objective of the present invention is to develop a more manufacturable method of the preparation of IL-2 formulations, which would allow to get a drug equal to well-known ones in terms of activity and therapeutic opportunities, that at the same time is suitable for large scale production and has low productivity costs.

The objective is solved by proposing a method of the preparation of IL-2 formulation, wherein yeast IL-2 producer cells are disrupted and target protein integrity is ensured and target protein (IL-2) is synthesized, and wherein the cells do not secrete the target protein and water insoluble fraction is separated while water insoluble compounds of disrupted cells are preserved in the formulation.

In particular, according to the present method, yeast producer is a strain of *Saccharomyces cerevisiae,* deposited as No. Y-791 in the All-Russian Collection of Industrial Microorganisms, which is a producer of human Interleukin-2 (IL-2).

Regardless of the existing opinion about necessity of careful purification of the recombinant protein, authors of the present invention have unexpectedly discovered that preservation of residues of yeast cell walls in recombinant IL-2 formulation not only permits to simplify its preparation scheme and obtain a cheaper drug, but also to obtain a highly effective drug whose therapeutic features in some aspects exceed a features of known IL-2 formulations.

Realization of the method for the preparation of the formulation without extraction (transfer) of IL-2 into liquid phase and its subsequent extraction from liquid phase leads to an unexpected result. The method results in an absolutely new IL-2 formulation instead of an under-purified one. Residuary traces in the formulation of insoluble fraction of yeast cell walls not only do not hinder effect of IL-2, but also act as a sort of adjuvant, i.e. subsidiary compound stimulating activation of T-cellular immunity in mammals. Transfer of water insoluble IL-2 into liquid phase with its subsequent extraction has been regarded as absolutely essential before the present invention, and efforts of scientists were directed at developing ways of purification improvement. After creation of the present invention it becomes apparent that IL-2 formulation must be prepared without removal of water insoluble fraction of yeast cell walls, and, further efforts can be concentrated in quality improvement of cell wall fraction itself.

In the formulation thus prepared IL-2 is inactive, which ensures its long-term storage. Solubilizer and water are to be added to the formulation to activate IL-2.

It is reasonable to disrupt cells in the presence of protease inhibitors as it is exactly the proteases released during disruption of yeast cells that target protein can be destroyed by.

Preferably, to disrupt cells using high speed disintegrator with glass beads in the presence of protease inhibitor as in this case cells are disrupted at a greater degree, while the target product is intact.

Preferably, water insoluble fraction after separation is washed for a more complete removal of water soluble components. This results in more complete removal of water soluble proteases and other yeast proteins, RNA, chromosomal and plasmid DNA.

Preferably, separated water insoluble fraction is dried. Drying can follow separation or rinsing.

More preferably, after separation, water insoluble fraction is treated with acetone with subsequent removal of acetone fraction. At that stage the disrupted yeast cells are dehydrated. This stage also permits to eliminate lipid components of water insoluble fraction which leads to preparing the formulation having enriched physical properties, convenient to utilize and better tolerated in therapeutic application.

While treating with acetone as mentioned above, a suspension of water insoluble fraction is prepared and then added to acetone at the volume ratio of suspension to acetone as 1:5 up to 1:20, preferably, 1:10, resulting suspension in acetone, then the suspension is filtered and the residue remained on the filter is dried. Before treatment with acetone water insoluble fraction can be washed, i.e. washing is done after separation of water insoluble fraction before preparation of its suspension in water.

In the preferable embodiment of the said method a dry solubilizer is added to the water insoluble fraction after its drying. In this way the formulation is prepared as a composition of dried water insoluble fraction and a solubilizer. The formulation thus prepared comprises IL-2 in inactive form, which ensures long-term storage thereof. To activate IL-2 water shall be added to such formulation. When water is added, a solubilizer concentration in the solution becomes optimal, which ensures formation of correct protein conformation and preparation of the biologically active formulation.

The use of sodium dodecyl sulphate as a solubilizer is advisable.

One of the embodiments of the method suggests that in a process of the preparation of IL-2 formulation some water is added after adding a solubilizer. This allows to prepare a formulation, wherein IL-2 is active protein with an optimal conformation and ready for immediate use.

According to the invention, IL-2 formulation is provided as any variant prepared by the above method. The formulation has lower production price and can be administered orally in therapy, which is more preferable for patients. It has been unexpectedly found out that such formulation demonstrates therapeutical effects that are exceed the ones typical for formulations based on purified IL-2 prepared by well known methods.

At the current stage of biotechnology and genetic engineering yeast producers of IL-2 are well known and can be obtained in depositaries or newly created.

Though *Saccharomyces cerevisiae* is the most widespread yeast producer, some other non-pathogenic yeasts can be such producers, e.g., *Saccharomyces bulardi,* or *Pichia* genus yeasts, e.g., *Pichia pastoris, Kluyveromyces* genus, e.g., *Kluyveromyces lactis,* or *Hansenula* genus, , e.g., *Hansenula polymorpha,* and the like. Method of generating recombinant plasmids containing IL-2 genes, in particular, human IL-2 gene, is widely known. The method of obtaining strains containing such recombinant plasmids is also well known.

Particularly, an example of yeast producers of human IL-2 is *Saccharomyces cerevisiae* yeast strain deposited as No. Y-791 in the All-Russian Collection of Industrial Microorganisms. This strain is a human IL-2 producer. Structure of the expression vector introduced into the producer strain is such that cells do not secret target proteins. Any other strain producing the heterologous IL-2 can also be used.

Optimal conditions under which IL-2 synthesis would occur are known for each producer. Synthesis of IL-2 may be constitutive or require induction or derepression depending on genetic design of the producer strain. In general terms, taking into account peculiarities of the selected strain, a specialist can select cultivation conditions that will result in obtaining the culture of yeast cells wherein IL-2 is synthesized.

Particularly, Y-791 strain is first grown with inhibiting of IL-2 synthesis in minimal mineral medium Sd1 with histidine to achieve optical density OD₆₀₀ from 2.5 to 4.0, more preferably, OD₆₀₀= 3.0. IL-2 synthesis is inhibited by presence of phosphate in the cultivation medium. The obtained inoculum is then subcultured in a greater volume of the second medium for cultivation, wherein IL-2 synthesis is derepressed and cultivated until the earlier stationary stage, i.e. optical density OD₆₀₀ ranging from 6.0 to 9.0, preferably OD₆₀₀= 7.5, is achieved. Medium Sd2 or medium PEP (containing 20 g/l of peptone and 20 g/l glucose) can be used as second media.

Upon completion of cultivation, cells can be separated from the cultural medium by centrifugation or filtration. Cells are further processed, while the cultural medium is disposed. When using Y-791 strain, one can normally obtain 8 - 12 g of cells per 1 liter of nutrient medium.

The obtained biomass can be used immediately or frozen in order to use it later in the proposed method.

It is noted that the preparation containing non-disrupted cells has just a trace activity in comparison to the preparation obtained from disrupted cells.

Yeast cells can be disrupted by any known methods that ensure integrity of target protein. In particular, by physical methods, for example, using a high-speed disintegrator with glass beads, under high pressure, by freezing-melting method. In this context, integrity of the target product is ensured by adding protease inhibitors to the suspension of disrupted cells, so that yeast proteases released resulting from disrupting of yeast cells should not destruct target protein, in particular, IL-2. Enzyme method of yeast cells disrupting is also possible if it is possible to avoid a proteolytic action of the used agent on the target protein.

Integrity of the target product is also ensured by applying this method at lowered temperatures ranging from +2 to +8°C, in particular, at +4°C.

Disintegrator DynoMill can be taken as an example of a high speed disintegrator with glass beads. It is preferable to disrupt cells at the temperature from +2°C to +10°C and at the centrifugation speed of 4000-6000 rpm.

PMSF (phenilmethylsulfonyl fluoride) is normally used as an inhibitor of yeast proteases, however, any other inhibitor or protease inhibitor group can be applied. It is sufficient to use 1mM of PMSF; however, any other appropriate amount of protease inhibitor can be used as determined basing on results of IL-2 content tests in the preparation by electrophoresis method in polyacrylamide gel.

Water insoluble fraction can be separated by centrifugation. Almost all water soluble components of yeast cells contents are transformed into supernatant fluid (supernatant), which is then disposed, and water insoluble components are precipitated. During centrifugation proteases, soluble proteins, ribonucleic acid (RNA), chromosome and plasmid DNA, etc. are disposed. Water insoluble fraction separation can be done by other available methods, in particular, by filtration.

It is appropriate to perform centrifugation at lowered temperatures, for example, at +4°C, and rotor rotation speed ranging from 4000 to 10000 rpm.

In the proposed method water insoluble fraction can be additionally processed for complete removal of water insoluble components, namely, by rinsing the fraction with a buffer solution. 0.005 M Tris-HCl, pH 7.2, buffer solution can be used as such buffer solution.

It is advisable to dry out the formulation. Formulation drying is provided to ensure opportunities for its storage and further use. The dried formulation contains the remainder of yeast cell walls with target proteins. This method excludes extraction stages of target protein from water insoluble fraction, which is considered to be an unexpected approach for this field of science as enormous efforts are normally concentrated precisely at this stage. All water insoluble components contained in the formulation do not prevent target properties manifestation under specific conditions, they sometimes contribute to them.

Freeze drying is normally used, but other appropriate drying methods can also be applicable.

The proposed method also provides an additional processing of water insoluble fraction with acetone. Before being processed with acetone, the water insoluble fraction may be washed with a buffer solution. Acetone processing includes the stages as follows. Water insoluble fraction is used for the preparation of suspension in water, which is then mixed with acetone by adding the suspension into acetone while stirring. Ratio of water suspension and acetone is from 1:5 to 1:20, preferably 1:10. The suspension should be sufficiently viscous, approximately 60-80%, preferably 75%. Acetone is processed at the temperature of -20°C. Dehydrated disrupted yeast cells are separated from acetone by vacuum filtration or centrifugation at the temperature ranging from -10°C to -30°C and rotation of 2000-5000 rpm. The obtained precipitate of disrupted yeast cells can be rinsed with twofold-tenfold (preferably, fivefold) volume of acetone and again separated from acetone by vacuum filtration or centrifugation at the temperature ranging from - 10°C to -30°C and rotation of 2000-5000 rpm. The precipitate is dried in an exhaust hood at room temperature for 20-30 hours. Acetone dehydrates preparation of cells, thus, freeze drying is not needed to obtain dry formulation.

According to embodiment of the method, the formulation is processed as a composition of dried water insoluble fraction of disrupted cells and dry solubilizer.

Solubilizer is a substance which facilitates dissolution of water insoluble substances in water. The recombinant IL-2 synthesized inside the yeast cell is water insoluble and linked to cell walls. It was found out that solubilizer also allows to separate IL-2 from cell walls. Sodium dodecyl sulphate, lauryl sarcosinate, deoxycholate, urea and Triton-X100 can be used as a solubilizer. If sodium dodecyl sulphate is used as a solubilizer, it is added in the amount of 4.0% - 20.0% (preferably 12%) of dry water insoluble fraction weight.

When IL-2 formulation is a composition of dried water insoluble fraction of disrupted cells and a solubilizer, IL-2 may be prepared for use by adding some water. It is enough to add 1-10 ml (preferably, 5 ml) of water to 100 mg of the formulation.

Dried preparation can be stored at a temperature of +4 to - 20°C for two years without adding special stabilizing agents or preservatives. Water should be added to the preparation shortly before use.

If it is necessary, stabilizers, antioxidants, excipients and other substances may be added to the formulation to impart a convenient pharmaceutical form to the drug.

The method may therefore be implemented in any of the following ways: disruption of yeast cells, separation of water insoluble fraction with its further drying; or otherwise, disruption of yeast cells, separation of water insoluble fraction, rinsing and drying this fraction; or otherwise, disruption of yeast cells, separation of water insoluble fraction with its possible further rinsing, processing with acetone with disposing the acetone fraction and drying of the precipitate; any of the above variants assumes that it is preferable after drying to make the preparation as a composition with dry solubilizer.

Biological activity of IL-2 formulation can be estimated by international standard test with the strain of IL-2-dependent tumor-specific cytotoxic mouse T-lymphocytes of CTLL-2 line (Hammerling U. et al. «Development and validation bioassay for interleukin-2». // J. Pharmaceut. & Biochem. Analysis, - 1992. - vol. 10. - p.547. Gearing A.J.H. and Thorpe R. «The international standard for human interleukin-2. Calibration by international collaborative study.» // J. Immunol. Methods. - 1984. - vol. 74. - p.39). Biologically active recombinant IL-2 should stimulate proliferation of these cells. Biological activity determination is provided by colorimetric method with tetrazolium dye MTT. The IL-2 stimulated viable cells absorb the dye and accumulate in cytoplasm water insoluble dark blue crystals of formazan (reduced MTT). After removal of cultural medium and complete dissolution of crystals in dimethyl sulfoxide, optical density of the obtained solution is measured by means of a computerized photometer at the wave length of 530 and 690 nm. Dark blue coloration of solution in samples containing IL-2 should depend on concentration of IL-2 introduced in the cultural medium. Comparing optical density of the sample and activity of standard of IL-2, biological activity of IL-2 in the incubatory sample is determined.

Quantity of IL-2 may be determined by any standard method used for determining the target protein, for example, by electrophoresis method in polyacrylamide gel (Laemmli U. "Cleavage of structural proteins during the assembly of the head of bacteriophage T4". // Nature. - 1970. - 227. - p. 680-685) or by using immune-enzyme analysis (Gehman L.O. and Robb R.J. "An ELISA-based assay for quantitation of human interleukin-2." // J. Immunol. Methods. - 1984. - vol.74. - p.39), etc.

In general terms, the method according to the invention can alternatively include, contain or substantially contain any appropriate stages described herein, and such invented method can additionally or alternatively exclude any stage or object that is used in a well-known method or which is not essential to achieve the objectives of this invention.

The same is applicable to the formulation according to the invention that may alternatively include, contain or substantially contain any appropriate components described herein, and such the formulation may be additionally or alternatively be prepared in such a way that any component, material, ingredient or object that is well known or is not essential to achieve the objectives of this invention can be excluded from it.

The invention is further illustrated by the following examples which do not restrict the scope of the invention.

### Example 1. Preparation of Interleukin-2 formulation

### 1.1. Preparation of IL-2 yeast producer biomass, wherein the target protein (IL-2) is synthesized

Aliquot of *Saccharomyces cerevisiae* Y-791 strain stored at -70°C in glycerin is inoculated in nutritional medium Sd1 containing 50 mg/l of histidin. Sdlmedium comprises 0.67% Yeast nitrogen base ("Difco Laboratories", Detroit, MI), 2% glucose. Ratio between cell mass and the volume of medium Sd1 is approximately 0.2 g to1 liter.

Cultivation in medium Sd1 is carried out until the optical density OD₆₀₀ ranging from 2.5 to 4.0 (preferably OD₆₀₀ =3.0) is achieved.

Upon reaching the required optical density value, the obtained inoculate is transferred in tenfold volume of the medium Sd2 and cultivated until OD₆₀₀ = 7.5 is achieved.

Upon completing the cultivation in medium Sd2, cells are separated from the cultural medium by means of centrifugation to obtain 10 g of cells from 1 liter of nutritional medium. All further manipulations are done with cells and the cultural medium is removed.

**Components of nutritional Sd2 medium**

| Medium component | Amount per 1 liter |
|---|---|
| L-Histidine | 0.05 g |
| (NH₄)- citrate (anhydrous) | 7.6 g |
| MgSO₄ | 0.5 g |
| NaCl | 0.1 g |
| CaCl₂x6H₂O | 0.1g |
| KCl | 1.5g |
| KH₂PO₄ | 0.03g |
| Glucose | 20g |
| H₃BO₃ | 0.5mg |
| CuSO₄x5H₂O | 0.04 mg |
| KI | 0.1 mg |
| FeCl₃x6H₂O | 0.2 mg |
| MnSO₄ | 0.4 mg |
| NaMoO₄x2H₂O | 0.2 mg |
| ZnSO₄x7H₂O | 0.4 mg |
| Biotin | 0.02 mg |
| Calcium pantothenate | 2 mg |
| Folic acid | 0.002 mg |
| Inositol | 10 mg |
| Nicotinic acid | 0.4 mg |
| P-benzaminic acid | 0.2 mg |
| Pyridoxine hydrochloride | 0.4 mg |
| Riboflavin | 0.2 mg |
| Thiamine | 0.4 mg |

### 1.2. Process of disruption of IL-2 yeast producer cells, wherein target protein (IL-2) is synthesized

A 50% suspension of cells with the buffer solution A is prepared by adding PMSF (phenilmethylsulfonyl fluoride) until the final concentration of 1 mM is obtained. Buffer solution A is a 0.005 M Tris-HCl buffer solution, pH 7.2. Yeast cells are disrupted by means of disintegrator Dyno Mill at the temperature ranging from +2 to +10°C and at speed of centrifugation 3000 rpm. Suspension of disrupted cells is collected in centrifugation bowls.

### 1.3. Separation of water insoluble fraction

The suspension obtained at stage 1.2. is centrifuged at the temperature of +4°C and speed of centrifugation 10000 rpm for 45 minutes. Supernatant liquid is removed.

### 1.4. Dry-out of water insoluble fraction

The precipitate is dried in freeze drying apparatus for 24 hours. From 1 g of cell culture, 100 mg of laminar glassy and rather fragile grey-fawn lyophilizate is yielded.

### Example 2. Preparation of Interleukin-2 formulation

Stages 1.1.-1.3 of Example 1 are carried out. The precipitate is then washed with 0.005 M Tris-HCl buffer solution, pH 7.2, after that the product is centrifuged at the temperature of +4°C and rotation speed of 10000 rpm for 45 minutes. Supernatant liquid is removed. The stage as described in 1.4 is further carried out.

### Example 3. Preparation of Interleukin-2 formulation

Stages 1.1.-1.3 of Example 1 are carried out. The precipitate is then washed with 0.005 M Tris-HCl buffer solution, pH 7.2, after that the product is again centrifuged at the temperature of +4°C and rotor rotation speed of 10000 rpm for 45 minutes. Supernatant liquid is removed.

75% suspension of the obtained precipitate of disrupted cells is prepared with water cooled down to +4°C, for example, 0.3 ml of water is added to 1 g of precipitate. The suspension is prepared on ice bath.

Glassware and acetone are cooled down to -10°C - -30°C for further manipulations. Ratio between volumes of suspension and acetone is 1:10, for example, 10 ml of acetone are required for 1 ml of suspension.

Suspension of disrupted yeast cells is slowly introduced into acetone and homogenized during 5 minutes.

Disrupted yeast cells are then separated from acetone by filtration at the temperature ranging from -10°C to -30°C.

The obtained precipitate of disrupted yeast cells is washed with fivefold volume of acetone and filtered again at the temperature ranging from -10°C to -30°C.

The precipitate is transferred into an enamel vessel and dried in an exhaust ventilation cabinet at room temperature for 18 hours.

Finally, of 10 g of wet yeast cells, 1 g of dry precipitate of disrupted yeast cells is obtained as fine homogenous whitish-fawn powder.

### Example 4. Addition of solubilizer

About 120 mg of sodium dodecyl sulphate (SDS) is added to 1 g of powder obtained in example 3 and mixed. It is essential to add some water to use the formulation prepared at this stage.

### Example 5. Addition of water

About 5 ml of water is added to 100 mg of the formulation prepared in Example 4 and mixed until a suspension is obtained.

### Example 6. Preparation of suspension to determine quantity and activity of IL-2

The suspension prepared in Example 5 is centrifuged for 5 minutes at 10000 rpm and supernatant is extracted. Precipitate is disposed. To analyze the obtained extract for specific activity and quantitative content of IL-2, it is necessary to take its aliquot, e.g. 10 µl.

Presence and quantity of IL-2 in the prepared extract is confirmed by electrophoresis, while its biological activity is proved by conventional test using CTLL-2 cell line.

### Example 7. Electrophoresis in polyacrylamide gel in the presence of sodium dodecyl sulphate

Apart from the samples of extract, test-solutions of bovine serum albumin are also added to the test for building a calibration curve in the amount of 1, 2, 4, 6 and 8 µg per line, as well as molecular weight markers for proteins 94, 67, 43, 30, 20.1 and 14.4 kDa.

Analysis of electrophoresis image proves presence of IL-2 (with a molecular weight of 15.3 kDa in comparison with the markers of proteins molecular weight) in the sample and allows to determine the amount of IL-2 using calibration curve for bovine serum albumin.

### Example 8. Biological activity of IL-2 formulation

Sample of IL-2 calibrated against an international reference reagent, stored at the temperature of -70°C and defrozen only once, is taken as a standard for determining activity of IL-2 being tested that is contained in the extract obtained in example 6.

About 100 µl of CTLL-2 cells rinsed with medium RPMI 1640 (1x10⁵ of living cells per ml) are added into each well with the standard solution and samples containing 10 µl of extract obtained in Example 6, and a plate is closed with non-tight lid. The solution is incubated at 37°C within 42 hours. In IL-2 containing samples CTLL-2 cells are proliferated. After incubation 10 µl of MTT solution is injected in each well and further incubated for additional 6 hours. Water insoluble dark blue crystals of formazan are formed in the viable cells stimulated by IL-2.

The plates are centrifuged within 5 minutes at 2000 rpm and supernatant is removed from the wells by sharp agitation of liquid. About 100 µl of dimethyl sulfoxide is added into each well and the plates are agitated with a shaker until the crystals of formazan are completely dissolved (for about 15 minutes).

Based on the spectrophotometric data of reference and tested samples, the activity of IL-2 in extract is calculated and its activity per 100 g of powder is estimated, total 4-5 million IU, at the average.

### Example 9. Treatment of dietary allergy with the formulation of Example 3

About 100 mg of the formulation of Example 4 is dissolved with 5 ml of water and administered by a patient suffering from green apple allergy. Treatment course lasted for 5 days according to the following regimen: contact with the allergic agent was scheduled every other day. The patient is administered the composition of the invention at the moment when allergic symptoms appeared. There were 3 contacts with green apples and 3 administrations of the formulation of the invention. On the 15^{th} day since the trial started the PRICK-test indicated absence of reaction to green apples. A check test performed in 6 months showed the same results.

Thus, the formulation of the invention has therefore proved its efficiency for treatment of the disease which has not yet been completely resolved until the present invention, because all the drugs known so far provided only symptomatic treatment. Treatment of dietary allergy by a number of known drugs based on the recombinant IL-2 has also not been provided, as it was supposed to be either ineffective or side effects of those drugs would outweigh the expected benefits.

### Example 10. Monotherapy of HIV-infections using the formulation of Example 4

The first two courses were conducted using 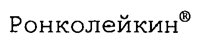 (Ronkoleukin^{®}) dry for injections (Biotech, Russia) representing purified protein; next two courses were conducted using the formulation prepared by the method of the invention and administered orally.

**CD4+ and HIV-viral Load Dynamics**

| Index | Before treatment | After two courses of Ronkoleukin^{®} | After the 3^{rd} course using the formulation of the invention | After the 4^{th} course using the formulation of the invention |
|---|---|---|---|---|
| CD4+ (units/ microliter) | 418 | 480 | 756 | 1066 |
| HIV (copies/ml) | 41000 | 20000 | 1500 | 950 |

Dynamics of both the first stage of treatment (using Ronkoleukin^{®}), and the second stage (using the formulation of the present invention) proved to be good.

Significant improvement of CD4+ contents dynamics when the formulation of the invention is used compared to pure IL-2 for injections may be caused by the reason that the formulation of the invention enhances proliferation of the immune system cells but also is able to activate them, because absence of activation factor sometimes results in absence of the effect of IL-2 injection.

Therefore, in addition to biologically active Interleukin-2, the formulation of the invention contains a number of adjuvants that allow to use this formulation when treatment with purified IL-2 is failed.

Further, the formulation is prepated in manufacturable way, method of preparation thereof does not require excessive time and special equipment. The formulation is active when administered orally.

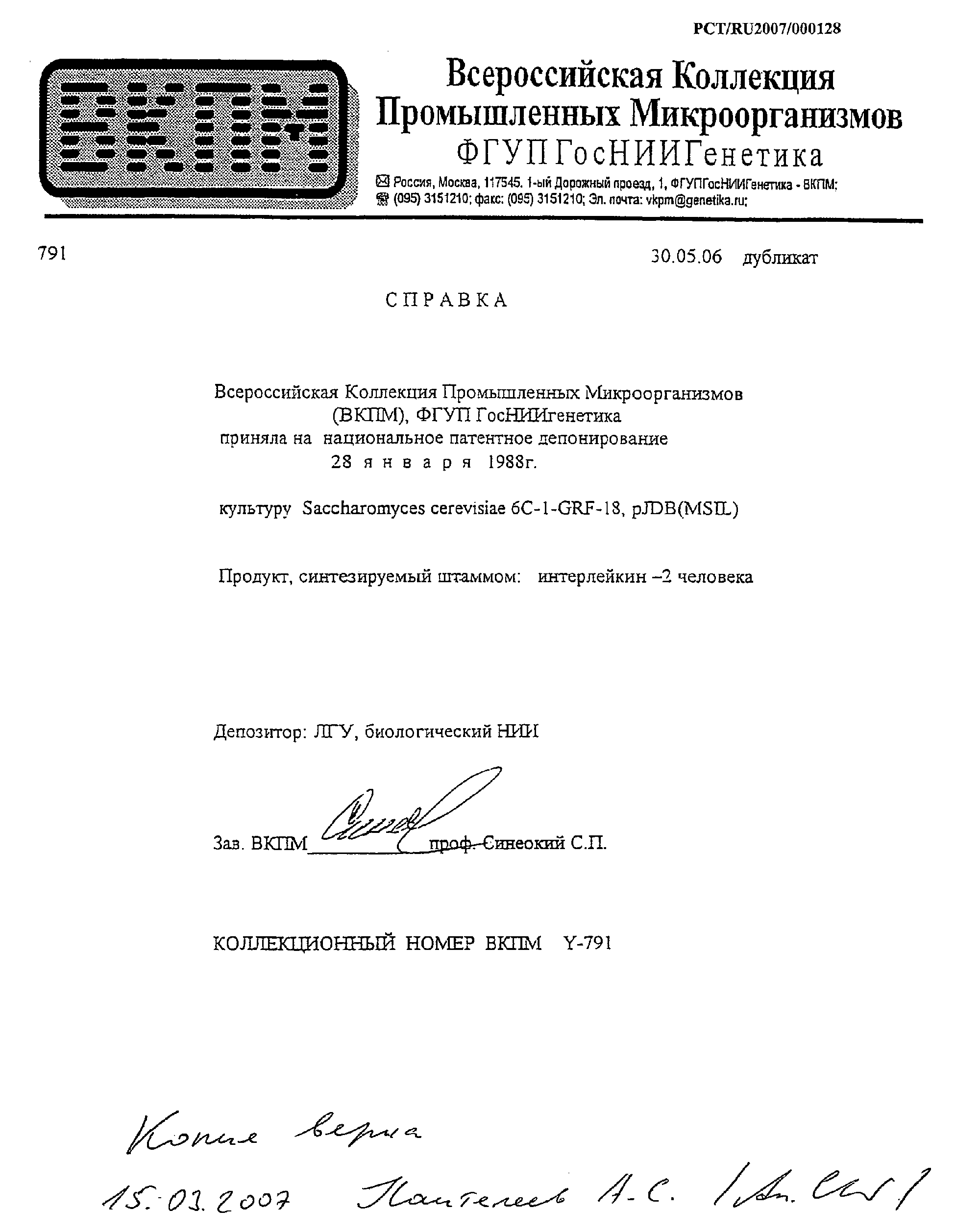
PCT/RU2007/000128


Всероссийская Коллекция
Промышленных Микроорганизмов
ФГУП ГосНИИГенетика

Россия, Москва, 117545. 1-ый Дорожный проезд, 1, ФГУПГосНИИГенетика - ВКПМ;
(095) 3151210; факс: (095) 3151210; Эл. почта: vkpm@genetika.ru;

791

30.05.06 дубликат

С П Р А В К А

Всероссийская Коллекция Промышленных Микроорганизмов
(ВКПМ), ФГУП ГосНИИгенетика
приняла на национальное патентное депонирование
28 я н в а р я 1988г.

культуру Saccharomyces cerevisiae 6C-1-GRF-18, pJDB(MSIL)

Продукт, синтезируемый штаммом: интерлейкин –2 человека

Депозитор: ЛГУ, биологический НИИ

Зав. ВКПМ [signature] проф. Синеокий С.П.

КОЛЛЕКЦИОННЫЙ НОМЕР ВКПМ Y-791

Копия верна
15.03.2007 Пантелеев А.С. /[signature]/

## Claims

1. A method for the preparation of interleukin-2 (IL-2) formulation, wherein IL-2 producer yeast cells, that synthesize the target protein (IL-2) and do not secrete said target protein, are disrupted and target protein integrity is ensured and wherein water insoluble fraction is separated and water insoluble compounds of disrupted cells are preserved in the formulation.

2. The method according to claim 1, **characterized in that** the yeast producer is a strain of *Saccharomyces cerevisiae,* deposited in All-Russian Collection of Industrial Microorganisms as No. Y-791 that is a producer of human interleukin-2.

3. The method according to claim 1, **characterized in that** the cells are disrupted in the presence of yeast protease inhibitor.

4. The method according to claim 1, **characterized in that** the cells are disrupted using a high-speed disintegrator with glass beads.

5. The method according to claim 1, **characterized in that** the separated water insoluble fraction is rinsed for a more complete removal of water soluble components.

6. The method according to claim 1, **characterized in that** the water insoluble fraction is dried out.

7. The method according to claim 1, **characterized in that** the separated water insoluble fraction is treated with acetone with further removal of acetone fraction.

8. The method according to claim 7, **characterized in that** after separation of water insoluble fraction suspension thereof in water is prepared and added into acetone with a ratio between water and acetone 1:5-1:20, thus obtaining a suspension in acetone, the obtained acetone suspension is then filtered and the precipitate left on filter is dried.

9. The method according to claim 8, **characterized in that** after separation of water insoluble fraction, prior to preparation of suspension thereof in water, the suspension is rinsed for a more complete removal of water soluble components.

10. The method according to claims 1-9, **characterized in that** after drying the separated water insoluble fraction, a dry solubilizer is added to the dry precipitate.

11. The method according to claim 10, **characterized in that** a solubilizer is sodium dodecyl sulphate.

12. The method according to claim 10, **characterized in that** after adding the solubilizer, water is added.

13. Interleukin-2 formulation prepared by any of the methods according to claims 1-12.
